Europäisches Patentamt

European Patent Office

Office européen des brevets

(10)

(11) Publication number: 0 076 620
B2

(12) NEW EUROPEAN PATENT SPECIFICATION

(45) Date of publication of the new patent specification: **31.10.90**

(21) Application number: **82305113.1**

(22) Date of filing: **28.09.82**

(51) Int. Cl.⁵: **C 07 D 473/12, A 23 F 5/20**

(54) Process for recovering caffeine from activated carbon.

(30) Priority: **28.09.81 US 306276**

(43) Date of publication of application:
**13.04.83 Bulletin 83/15**

(45) Publication of the grant of the patent:
**21.08.85 Bulletin 85/34**

(45) Mention of the opposition decision:
**31.10.90 Bulletin 90/44**

(84) Designated Contracting States:
**AT BE CH DE FR GB IT LI LU NL SE**

(56) References cited:
**EP-A-0 042 295**
**DD-A- 78 586**
**GB-A-1 488 340**

**N.A. Izmailov, "Fundamentals of Adsorption Technology of Separating Substances from Solutions", USPEKHI KHIMII, Vol. 24, Issue 3, 1955 pp. 346-376**
**J. Am. Soc. 29, pp. 1081-1091**

**The file contains technical information submitted after the application was filed and not included in this specification**

(73) Proprietor: **GENERAL FOODS CORPORATION**
**250 North Street**
**White Plains, N.Y. 10625 (US)**

(72) Inventor: **Katz, Saul Norman**
**7, Dorchester Drive**
**Monsey New York 10952 (US)**
Inventor: **Proscia, George Edward**
**135, Hilary Street**
**West Sayville New York 11796 (US)**

(74) Representative: **Baillie, Iain Cameron et al**
**c/o Ladas & Parry Isartorplatz 5**
**D-8000 München 2 (DE)**

Courier Press, Leamington Spa, England.

## Description

Technical Field

The present invention relates to decaffeination, and particularly to an improved process for recovering caffeine from an activated carbon adsorbent.

The decaffeination of vegetable materials and vegetable material extracts is of major commercial importance. Also significant is the recovery and sale of the caffeine removed from vegetable sources such as coffee and tea. It is known that activated carbon is a good adsorbent in caffeine recovery and purification procedures, but the carbon tends to hold the caffeine so tenaciously that, often, significant quantities of caffeine are lost or reduced in commercial value. Moreover, the carbon itself has commercial value since if it is regenerated it may again be employed in caffeine recovery and purification processes. The techniques currently available to the art for separating the caffeine from the carbon have not been wholly satisfactory in terms of both degree and quality of caffeine recovery. Further, inefficient separation of caffeine from the carbon results in difficulties in carbon regeneration.

Background Art

The recovery of caffeine from decaffeinating solvents has been an active area of concern for many years. For example, in U.S. 2,508,545, Shuman discloses that activated carbon and other adsorbents had been used to remove impurities from solutions of caffeine extracted from coffee. Shuman indicates that until the time of his invention caffeine losses due to adsorption onto the carbon ran as high as 10 to 14%. To rectify this, Shuman disclosed alternate use of organic and aqueous extractions with the final aqueous extraction being done at a pH of at least 7. While activated carbon is employed to remove impurities from the aqueous extract, the amounts employed are apparently small and no mention of separating caffeine from the carbon is made. Similarly, in U.S. 2,472,881, Bender employs activated carbon to remove impurities from an aqueous caffeine solution but does not discuss the steps taken to recover the caffeine adsorbed on the carbon.

Recently, an improved decaffeination method was disclosed by Vitzthum et al. in U.S. 3,879,569 wherein quantitative extraction of caffeine from raw coffee beans is achieved with moist supercritical carbon dioxide. This process produces a caffeine containing carbon dioxide stream from which essentially all of the caffeine can be removed by activated carbon. Unfortunately, the prior art techniques do not economically provide good levels of recovery of caffeine from the activated carbon or they require the use of chlorinated hydrocarbon solvents which are otherwise avoided by the use of carbon dioxide as an extractant.

In specification DD-A-785586 (H. Krahnefeld) there is disclosed use of dilute acids for the extraction of caffeine which acids include dilute acetic acid or dilute sulfuric acid. Such dilute acids are relatively inefficient in the removal of caffeine. In our copending European Patent Application No. 81302694.5 (EP-A-42295) we describe and claim a process for recovering caffeine from activated carbon comprising contacting the carbon with an organic acid or an alcohol. Acetic acid and acetic acid azeotropes are especially preferred as the solvents. However, when glacial acetic acid is employed operational and safety problems arise because of the low (40°C) flashpoint of glacial acetic acid and of its causing burns of the skin.

Based on economy, effect of final product quality and effectiveness, glacial acetic acid is the most efficient of all the solvents. It will be appreciated, however, that care must be taken when using glacial acetic acid due to its low flashpoint in the order of 40°C—43.3°C (open cut test) and burn causing properties well known in the literature for example in The Condensed Chemical Dictionary-Rose-Reinhold Publishing Corporation 1966.

Disclosure of Invention

The present invention now enables the recovery of caffeine from an activated carbon adsorbent by an improved process which comprises: contacting activated carbon having caffeine adsorbed thereon with an aqueous acetic acid solution, having a concentration of between about 50 and 80%, by weight, and most preferably having a concentration of about 70%, by weight, and which is capable of displacing at least a portion of the caffeine from active sites on the carbon; maintaining the contact for a period of time and at a temperature effective for the acetic acid solution to displace at least a portion of the caffeine from the carbon and dissolve the displaced caffeine; and separating the caffeine from the acetic acid solution.

This invention is based on the discovery that acetic acid water mixtures, having a concentration of between about 50% and 80%, by weight, may be employed to elute either batchwise or continuously in a counter-current manner the caffeine and other solids from the spent activated carbon. Although acetic acid-water mixtures are usually less efficient than pure glacial acetic acid they offer the advantage of being totally nonflammable with no flashpoint. Glacial acetic acid is flammable with a flashpoint of 104°F. Flammable solvents require equipment that is explosion-proof and proper safe buildings. These requirements significantly increase the capital costs for the process. By adding water to glacial acetic acid or using acetic acid-water mixtures this requirement is eliminated and thereby dramatically reduces capital costs. Preferably, acetic acid of 70% concentration which is commercially available is used.

The present invention also takes advantage of the discovery that acetic acid has the ability to effect desorption both by its strong solvent ability and its ability to displace the adsorbed material from active sites on the adsorbent and is extremely effective in separating caffeine from activated carbon. The strong solvent effect causes a partitioning between the activated carbon and the acetic acid. The site displacement effect is achieved by the acetic acid molecules themselves competing for the active adsorbent sites. Once a caffeine molecule is displaced, it is then taken into solution by the acetic acid solution which is a strong solvent for caffeine.

The acetic acid solution is employed according to the present invention at temperatures in excess of 100°C to obtain the greatest rates of recovery of caffeine. Temperature has a strong effect on desorption and should therefore be as high as possible, consistent with maintaining the acetic acid solution as a liquid. Where temperatures higher than the boiling point of the acetic acid solution are desired, it will be necessary to employ pressures in excess of atmospheric.

Because the caffeine is valuable for food and pharmaceutical use, the acetic acid must be food-grade. The acetic acid solution is liquid at the proposed processing temperature and at room temperature, is an excellent solvent for caffeine, is capable of displacing caffeine from the active sites on the carbon and is an especially effective solvent for use according to the invention since it eliminates the flammability problem which may arise with glacial acetic acid. The acetic acid solution has a concentration of about 50 to 80%, and preferably 70%, by weight.

The acetic acid solution is maintained in contact with the carbon having caffeine adsorbed thereon for a period of time and at a temperature effective for the solvent to displace at least a portion of the caffeine from the carbon and dissolve the displaced caffeine. As noted above, preferred temperatures will be above 100°C, but the specific temperature for any particular process will be selected on its own set of economic considerations and may be below this. Practical contact times will be determined on the basis of the desired degree of recovery and the desorption rate for a particular system. Preferably, the contact time should be sufficient to permit displacement (recovery) of at least 80% by weight of the caffeine from the carbon and into solution with the acetic acid solution. Because the caffeine is valuable as a product and, if not removed from the carbon, decreases the adsorbent capacity of the carbon, higher rates of displacement, on the order of 90% by weight or more, are desired.

As the activated carbon adsorbent can be any of those types commercially available which are effective caffeine adsorbents and capable of withstanding the rigors of recycling permitted by the invention. Preferred activated carbons are those prepared from coconut, coal and lignite, particularly those available commercially from Calgon Corporation, ICI, Carborundum and Union Carbide Corporation.

After contact for the requisite period of time, the activated carbon is preferably separated from the acetic acid solution prior to separation of the caffeine from the solvent. The simplest and most effective manner for removing the carbon from the solution is by filtration.

The caffeine may be converted to U.S.P. caffeine by techniques of dissolution, crystallization, solvent purification and the like.

The activated carbon may be regenerated by washing and steaming to remove residual acetic acid. The activated carbon may then be reused through several more decaffeination cycles before reactivation, usually by means of thermal reactivation, is required.

Best Mode for Carrying Out the Invention

The following example is for the purpose of illustrating and explaining the best mode for carrying out the invention, but is not meant to be limiting in any regard. Unless otherwise indicated, all parts and percentages are by weight.

Example 1

One hundred parts of 70% acetic acid solution and 10 parts of activated carbon pellets containing 9.1% by weight caffeine, obtained from the process of U.S. 3,879,569, of Vitzthum et al. were introduced into a Soxhlet extraction tube and then refluxed at atmospheric pressure for 16 hours. The caffeine was recovered from the solution by evaporation. The total amount of caffeine recovered was 0.91 parts, 100% recovery.

**Claims**

1. A process for recovering caffeine adsorbed on activated carbon from vegetable source solutions comprising:

contacting activated carbon having caffeine adsorbed thereon with an aqueous acetic acid solution having a concentration from 50% to 80% by weight and which is capable of displacing at least a portion of the caffeine from active sites on the carbon;

maintaining the contact for a period of time and at a temperature effective for the solution to displace at least a portion of the caffeine from the carbon and dissolve the displaced caffeine; and

separating the carbon from the solution.

2. A process according to claim 1 wherein the contact is maintained at a temperature of at least 100°C.

3. A process according to either of claims 1 and 2 wherein the contact is maintained at a pressure in excess of atmospheric.

4. A process according to any one of claims 1 to 3 wherein contact is maintained for a period of time sufficient to permit displacement of at least 80% by weight of the caffeine from the carbon and into solution with the acetic acid solution.

5. A process according to any one of claims 1 to 4 wherein the carbon is separated from the solution by decantation prior to separating the caffeine from the solution.

6. A process according to claim 5 wherein the caffeine is separated from the solution by steam distillation.

7. A process according to claim 5 wherein the caffeine is separated from the solution by evaporating the solution.

8. A process according to any one of claims 1 to 7 wherein the caffeine is further purified and refined and the carbon is regenerated and/or reactivated.

**Patentansprüche**

1. Verfahren zum Gewinnen von Koffein, das aus pflanzlichem Ausgangsgut erhaltenen Lösungen auf Aktivkohle adsorbiert ist, in dem adsorbiertes Koffein enthaltende Aktivkohle mit einer wässerigen Essigsäurelösung in Berührung gebracht wird, die eine Konzentration von 50 bis 80 Gew.% hat und geeignet ist, mindestens einen Teil des Koffeins von aktiven Bindungsplätzen der Kohle zu verdrängen,

die Berührung so lange und bei einer solchen Temperatur aufrechterhalten wird, daß die Lösung mindestens einen Teil des Koffeins von der Kohle verdrängt und das verdrängte Koffein auflöst und

die Kohle von der Lösung abgetrennt wird.

2. Verfahren nach Anspruch 1, dadurch gekennzeichnet, daß die Berührung bei einer Temperatur von mindestens 100°C aufrechterhalten wird.

3. Verfahren nach Anspruch 1 oder 2, dadurch gekennzeichnet, daß die Berührung unter einem Überdruck aufrechterhalten wird.

4. Verfahren nach einem der Ansprüche 1 bis 3, dadurch gekennzeichnet, daß die Berührung so lange aufrechterhalten wird, daß mindestens 80 Gew.% des Koffeins von der Kohle verdrängt und in der Essigsäurelösung gelöst werden.

5. Verfahren nach einem der Ansprüche 1 bis 4, dadurch gekennzeichnet, daß die Kohle von der Lösung durch Dekantation abgetrennt wird, ehe das Koffein von der Lösung abgetrennt wird.

6. Verfahren nach Anspruch 5, dadurch gekennzeichnet, daß das Koffein von der Lösung durch Dampfdestillation abgetrennt wird.

7. Verfahren nach Anspruch 5, dadurch gekennzeichnet, daß das Koffein von der Lösung durch Verdampfen der Lösung abgetrennt wird.

8. Verfahren nach einem der Ansprüche 1 bis 7, dadurch gekennzeichnet, daß das Koffein weiter gereinigt und raffiniert und die Kohle regeneriert und/oder reaktiviert wird.

**Revendications**

1. Procédé de récupération de caféine adsorbée sur du charbon actif à partir de solutions de source végétale comprenant:

la mise en contact de charbon actif sur lequel est présente de la caféine adsorbée, avec une solution aqueuse d'acide acétique ayant une concentration d'environ 50 à 80% en poids et capable de déplacer une partie au moins de la caféine à partir d'emplacements actifs du charbon;

le maintien du contact pendant un temps et à une température permettant à la solution de déplacer au moins une partie de la caféine à partir du charbon et de dissoudre la caféine déplacée; et

la séparation de la caféine d'avec la solution.

2. Procédé selon la revendication 1, caractérisé en ce qu'on maintient le contact à température d'au moins 100°C.

3. Procédé selon les revendications 1 et 2, caractérisé en ce qu'on maintient le contact sous une pression supérieure à la pression atmosphérique.

4. Procédé selon l'une quelconque des revendications 1 à 3, caractérisé en ce qu'on maintient le contact pendant un laps de temps suffisant pour permettre le déplacement d'au moins 80% en poids de la caféine à partir du charbon et sa dissolution dans la solution d'acide acétique.

5. Procédé selon l'une quelconque des revendications 1 à 4, caractérisé en ce qu'on sépare le charbon de la solution par décantation avant de séparer la caféine de la solution.

6. Procédé selon la revendication 5, caractérisé en ce qu'on sépare la caféine de la solution par distillation à la vapeur d'eau.

7. Procédé selon la revendication 5, caractérisé en ce qu'on sépare la caféine de la solution par évaporation de la solution.

8. Procédé selon l'une quelconque des revendications 1 à 7, caractérisé en ce que la caféine est encore purifiée et raffinée et le charbon est régénéré et/ou réactivé.